(19) **Europäisches Patentamt European Patent Office Office européen des brevets**

(11) **EP 1 755 656 B1**

(12) **EUROPEAN PATENT SPECIFICATION**

(45) Date of publication and mention of the grant of the patent:
**07.07.2010 Bulletin 2010/27**

(21) Application number: **05744343.4**

(22) Date of filing: **24.05.2005**

(51) Int Cl.:
*A61K 38/48* (2006.01)    *A61P 1/18* (2006.01)
*A61P 3/10* (2006.01)    *A61P 11/00* (2006.01)
*A61P 1/14* (2006.01)

(86) International application number:
**PCT/DK2005/000342**

(87) International publication number:
**WO 2005/115445 (08.12.2005 Gazette 2005/49)**

(54) **ENZYMES FOR PHARMACEUTICAL USE**

ENZYME FÜR PHARMAZEUTISCHE ANWENDUNG

ENZYMES A VOCATION PHARMACEUTIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **24.05.2004 DK 200400810**
**20.01.2005 DK 200500101**

(43) Date of publication of application:
**28.02.2007 Bulletin 2007/09**

(60) Divisional application:
**09155524.3**

(73) Proprietor: **Novozymes A/S**
**2880 Bagsvaerd (DK)**

(72) Inventors:
• **SVENDSEN, Allan**
**DK-2970 Hørsholm (DK)**
• **KAASGAARD, Svend**
**DK-2740 Skovlunde (DK)**

• **BORCH, Kim**
**DK-3460 Birkerød (DK)**
• **FISCHER, Morten**
**DK-2950 Vedbæk (DK)**
• **PETTERSSON, Dan**
**DK-3540 Lynge (DK)**
• **GREGORY, Peter, Colin**
**30559 Hannover (DE)**

(56) References cited:
**WO-A-00/54799    WO-A-01/58276**
**WO-A-97/37681    WO-A-02/060474**
**WO-A-03/106667**

• **LEBENTHAL E ET AL: "ENZYME THERAPY FOR PANCREATIC INSUFFICIENCY: PRESENT STATUS AND FUTURE NEEDS" PANCREAS, RAVEN PRESS, NEW YORK, NY, US, vol. 9, no. 1, 1994, pages 1-12, XP001024461 ISSN: 0885-3177**

SEQUENCE LISTING

<110> Novozymes A/S
<120> Enzymes for Pharmaceutical use
<130> 10627.204-wo
<160> 9
<170> PatentIn version 3.2
<210> 1
<211> 188
<212> PRT
<213> Nocardiopsis sp. NRRL 18262
<220>
<221> mat_peptide

<222> (1)..(188)
<400> 1

```
Ala Asp Ile Ile Gly Gly Leu Ala Tyr Thr Met Gly Gly Arg Cys Ser
1               5                   10                  15

Val Gly Phe Ala Ala Thr Asn Ala Ala Gly Gln Pro Gly Phe Val Thr
            20                  25                  30

Ala Gly His Cys Gly Arg Val Gly Thr Gln Val Thr Ile Gly Asn Gly
        35                  40                  45

Arg Gly Val Phe Glu Gln Ser Val Phe Pro Gly Asn Asp Ala Ala Phe
    50                  55                  60

Val Arg Gly Thr Ser Asn Phe Thr Leu Thr Asn Leu Val Ser Arg Tyr
65                  70                  75                  80

Asn Thr Gly Gly Tyr Ala Thr Val Ala Gly His Asn Gln Ala Pro Ile
                85                  90                  95

Gly Ser Ser Val Cys Arg Ser Gly Ser Thr Thr Gly Trp His Cys Gly
            100                 105                 110

Thr Ile Gln Ala Arg Gly Gln Ser Val Ser Tyr Pro Glu Gly Thr Val
        115                 120                 125

Thr Asn Met Thr Arg Thr Thr Val Cys Ala Glu Pro Gly Asp Ser Gly
    130                 135                 140

Gly Ser Tyr Ile Ser Gly Thr Gln Ala Gln Gly Val Thr Ser Gly Gly
145                 150                 155                 160

Ser Gly Asn Cys Arg Thr Gly Gly Thr Thr Phe Tyr Gln Glu Val Thr
                165                 170                 175

Pro Met Val Asn Ser Trp Gly Val Arg Leu Arg Thr
                180                 185
```

<210> 2
<211> 188
<212> PRT
<213> Nocardiopsis dassonvillei subsp. dassonvillei DSM 43235
<220>
<221> mat_peptide
<222> (1)..(188)
<400> 2

```
Ala Asp Ile Ile Gly Gly Leu Ala Tyr Tyr Met Gly Gly Arg Cys Ser
1               5                   10                  15

Val Gly Phe Ala Ala Thr Asn Ser Ala Gly Gln Pro Gly Phe Val Thr
            20              25              30

Ala Gly His Cys Gly Thr Val Gly Thr Gly Val Thr Ile Gly Asn Gly
        35              40              45

Thr Gly Thr Phe Gln Asn Ser Val Phe Pro Gly Asn Asp Ala Ala Phe
    50              55              60

Val Arg Gly Thr Ser Asn Phe Thr Leu Thr Asn Leu Val Ser Arg Tyr
65              70              75              80

Asn Ser Gly Gly Tyr Gln Ser Val Thr Gly Thr Ser Gln Ala Pro Ala
            85              90              95

Gly Ser Ala Val Cys Arg Ser Gly Ser Thr Thr Gly Trp His Cys Gly
            100             105             110

Thr Ile Gln Ala Arg Asn Gln Thr Val Arg Tyr Pro Gln Gly Thr Val
        115             120             125

Tyr Ser Leu Thr Arg Thr Asn Val Cys Ala Glu Pro Gly Asp Ser Gly
    130             135             140

Gly Ser Phe Ile Ser Gly Ser Gln Ala Gln Gly Val Thr Ser Gly Gly
145             150             155             160

Ser Gly Asn Cys Ser Val Gly Gly Thr Thr Tyr Tyr Gln Glu Val Thr
            165             170             175

Pro Met Ile Asn Ser Trp Gly Val Arg Ile Arg Thr
            180             185
```

<210> 3
<211> 188
<212> PRT
<213> Nocardiopsis alba DSM 15647
<220>
<221> mat_peptide
<222> (1)..(188)
<400> 3

```
Ala Asp Ile Ile Gly Gly Leu Ala Tyr Thr Met Gly Gly Arg Cys Ser
1               5                   10                  15

Val Gly Phe Ala Ala Thr Asn Ala Ser Gly Gln Pro Gly Phe Val Thr
            20              25                  30

Ala Gly His Cys Gly Thr Val Gly Thr Pro Val Ser Ile Gly Asn Gly
        35              40                  45

Gln Gly Val Phe Glu Arg Ser Val Phe Pro Gly Asn Asp Ser Ala Phe
    50              55                  60

Val Arg Gly Thr Ser Asn Phe Thr Leu Thr Asn Leu Val Ser Arg Tyr
65              70                  75                  80

Asn Thr Gly Gly Tyr Ala Thr Val Ser Gly Ser Ser Gln Ala Ala Ile
            85                  90                  95

Gly Ser Gln Ile Cys Arg Ser Gly Ser Thr Thr Gly Trp His Cys Gly
            100                 105                 110

Thr Val Gln Ala Arg Gly Gln Thr Val Ser Tyr Pro Gln Gly Thr Val
        115             120                 125

Gln Asn Leu Thr Arg Thr Asn Val Cys Ala Glu Pro Gly Asp Ser Gly
    130             135                 140

Gly Ser Phe Ile Ser Gly Ser Gln Ala Gln Gly Val Thr Ser Gly Gly
145             150                 155                 160

Ser Gly Asn Cys Ser Phe Gly Gly Thr Thr Tyr Tyr Gln Glu Val Asn
            165                 170                 175

Pro Met Leu Ser Ser Trp Gly Leu Thr Leu Arg Thr
            180             185
```

<210> 4
<211> 188
<212> PRT
<213> Nocardiopsis prasina DSM 15648
<220>
<221> mat_peptide
<222> (1)..(188)
<400> 4

```
Ala Asp Ile Ile Gly Gly Leu Ala Tyr Thr Met Gly Gly Arg Cys Ser
1               5                   10                  15

Val Gly Phe Ala Ala Thr Asn Ala Ala Gly Gln Pro Gly Phe Val Thr
            20              25                  30

Ala Gly His Cys Gly Arg Val Gly Thr Gln Val Ser Ile Gly Asn Gly
        35              40                  45

Gln Gly Val Phe Glu Gln Ser Ile Phe Pro Gly Asn Asp Ala Ala Phe
    50              55                  60

Val Arg Gly Thr Ser Asn Phe Thr Leu Thr Asn Leu Val Ser Arg Tyr
65              70                  75                  80

Asn Thr Gly Gly Tyr Ala Thr Val Ala Gly His Asn Gln Ala Pro Ile
            85              90                  95

Gly Ser Ser Val Cys Arg Ser Gly Ser Thr Thr Gly Trp His Cys Gly
            100             105                 110

Thr Ile Gln Ala Arg Gly Gln Ser Val Ser Tyr Pro Glu Gly Thr Val
        115             120                 125

Thr Asn Met Thr Arg Thr Thr Val Cys Ala Glu Pro Gly Asp Ser Gly
    130             135                 140

Gly Ser Tyr Ile Ser Gly Asn Gln Ala Gln Gly Val Thr Ser Gly Gly
145             150                 155                 160

Ser Gly Asn Cys Arg Thr Gly Gly Thr Thr Phe Tyr Gln Glu Val Thr
            165             170                 175

Pro Met Val Asn Ser Trp Gly Val Arg Leu Arg Thr
            180             185
```

<210> 5
<211> 188
<212> PRT
<213> Nocardiopsis prasina DSM 15649
<220>
<221> mat_peptide
<222> (1)..(188)
<400> 5

```
Ala Asp Ile Ile Gly Gly Leu Ala Tyr Thr Met Gly Gly Arg Cys Ser
1               5                   10                  15

Val Gly Phe Ala Ala Thr Asn Ala Ala Gly Gln Pro Gly Phe Val Thr
            20              25                  30
```

Ala Gly His Cys Gly Arg Val Gly Thr Gln Val Thr Ile Gly Asn Gly
     35           40           45

Arg Gly Val Phe Glu Gln Ser Ile Phe Pro Gly Asn Asp Ala Ala Phe
    50           55          60

Val Arg Gly Thr Ser Asn Phe Thr Leu Thr Asn Leu Val Ser Arg Tyr
65          70           75          80

Asn Thr Gly Gly Tyr Ala Thr Val Ala Gly His Asn Gln Ala Pro Ile
         85          90          95

Gly Ser Ser Val Cys Arg Ser Gly Ser Thr Thr Gly Trp His Cys Gly
       100        105       110

Thr Ile Gln Ala Arg Gly Gln Ser Val Ser Tyr Pro Glu Gly Thr Val
    115         120         125

Thr Asn Met Thr Arg Thr Thr Val Cys Ala Glu Pro Gly Asp Ser Gly
    130         135        140

Gly Ser Tyr Ile Ser Gly Asn Gln Ala Gln Gly Val Thr Ser Gly Gly
145         150         155        160

Ser Gly Asn Cys Arg Thr Gly Gly Thr Thr Phe Tyr Gln Glu Val Thr
       165        170       175

Pro Met Val Asn Ser Trp Gly Val Arg Leu Arg Thr
       180        185

<210> 6
<211> 196
<212> PRT
<213> Synthetic
<220>
<221> mat_peptide
<222> (1)..(196)
<400> 6

Ala Asp Ile Ile Gly Gly Leu Ala Tyr Tyr Met Gly Gly Arg Cys Ser
1          5          10          15

Val Gly Phe Ala Ala Thr Asn Ala Ser Gly Gln Pro Gly Phe Val Thr
       20         25        30

Ala Gly His Cys Gly Thr Val Gly Thr Pro Val Ser Ile Gly Asn Gly
     35           40          45

Lys Gly Val Phe Glu Arg Ser Ile Phe Pro Gly Asn Asp Ser Ala Phe
    50           55          60

Val Arg Gly Thr Ser Asn Phe Thr Leu Thr Asn Leu Val Ser Arg Tyr
65                    70              75              80

Asn Ser Gly Gly Tyr Ala Thr Val Ala Gly His Asn Gln Ala Pro Ile
                85                  90                  95

Gly Ser Ala Val Cys Arg Ser Gly Ser Thr Thr Gly Trp His Cys Gly
            100                 105                 110

Thr Ile Gln Ala Arg Asn Gln Thr Val Arg Tyr Pro Gln Gly Thr Val
        115                 120                 125

Tyr Ser Leu Thr Arg Thr Thr Val Cys Ala Glu Pro Gly Asp Ser Gly
    130                 135                 140

Gly Ser Tyr Ile Ser Gly Thr Gln Ala Gln Gly Val Thr Ser Gly Gly
145                 150                 155                 160

Ser Gly Asn Cys Ser Ala Gly Gly Thr Thr Tyr Tyr Gln Glu Val Asn
            165                 170                 175

Pro Met Leu Ser Ser Trp Gly Leu Thr Leu Arg Thr Gln Ser His Val
            180                 185                 190

Gln Ser Ala Pro
        195

<210> 7
<211> 8
<212> PRT
<213> Synthetic
<220>
<221> MISC_FEATURE
<222> (1)..(8)
<223> C-terminal extension
<400> 7

Gln Ser His Val Gln Ser Ala Pro
1               5

<210> 8
<211> 269
<212> PRT
<213> Thermomyces lanuginosus
<220>
<221> mat_peptide
<222> (1)..()
<400> 8

Glu Val Ser Gln Asp Leu Phe Asn Gln Phe Asn Leu Phe Ala Gln Tyr
1               5                   10                  15

```
Ser Ala Ala Ala Tyr Cys Gly Lys Asn Asn Asp Ala Pro Ala Gly Thr
            20              25              30

Asn Ile Thr Cys Thr Gly Asn Ala Cys Pro Glu Val Glu Lys Ala Asp
        35              40              45

Ala Thr Phe Leu Tyr Ser Phe Glu Asp Ser Gly Val Gly Asp Val Thr
    50              55              60

Gly Phe Leu Ala Leu Asp Asn Thr Asn Lys Leu Ile Val Leu Ser Phe
65              70              75              80

Arg Gly Ser Arg Ser Ile Glu Asn Trp Ile Gly Asn Leu Asn Phe Asp
            85              90              95

Leu Lys Glu Ile Asn Asp Ile Cys Ser Gly Cys Arg Gly His Asp Gly
            100             105             110

Phe Thr Ser Ser Trp Arg Ser Val Ala Asp Thr Leu Arg Gln Lys Val
        115             120             125

Glu Asp Ala Val Arg Glu His Pro Asp Tyr Arg Val Val Phe Thr Gly
    130             135             140

His Ser Leu Gly Gly Ala Leu Ala Thr Val Ala Gly Ala Asp Leu Arg
145             150             155             160

Gly Asn Gly Tyr Asp Ile Asp Val Phe Ser Tyr Gly Ala Pro Arg Val
            165             170             175

Gly Asn Arg Ala Phe Ala Glu Phe Leu Thr Val Gln Thr Gly Gly Thr
            180             185             190

Leu Tyr Arg Ile Thr His Thr Asn Asp Ile Val Pro Arg Leu Pro Pro
        195             200             205

Arg Glu Phe Gly Tyr Ser His Ser Ser Pro Glu Tyr Trp Ile Lys Ser
    210             215             220

Gly Thr Leu Val Pro Val Thr Arg Asn Asp Ile Val Lys Ile Glu Gly
225             230             235             240

Ile Asp Ala Thr Gly Gly Asn Asn Gln Pro Asn Ile Pro Asp Ile Pro
            245             250             255

Ala His Leu Trp Tyr Phe Gly Leu Ile Gly Thr Cys Leu
            260             265
```

<210> 9
<211> 478
<212> PRT

<213> Aspergillus oryzae

<220>

<221> mat_peptide

<222> (1)..(478)

<400> 9

```
Ala Thr Pro Ala Asp Trp Arg Ser Gln Ser Ile Tyr Phe Leu Leu Thr
1               5                   10                  15

Asp Arg Phe Ala Arg Thr Asp Gly Ser Thr Thr Ala Thr Cys Asn Thr
            20                  25                  30

Ala Asp Gln Lys Tyr Cys Gly Gly Thr Trp Gln Gly Ile Ile Asp Lys
        35                  40                  45

Leu Asp Tyr Ile Gln Gly Met Gly Phe Thr Ala Ile Trp Ile Thr Pro
    50                  55                  60

Val Thr Ala Gln Leu Pro Gln Thr Thr Ala Tyr Gly Asp Ala Tyr His
65                  70                  75                  80

Gly Tyr Trp Gln Gln Asp Ile Tyr Ser Leu Asn Glu Asn Tyr Gly Thr
                85                  90                  95

Ala Asp Asp Leu Lys Ala Leu Ser Ser Ala Leu His Glu Arg Gly Met
            100                 105                 110

Tyr Leu Met Val Asp Val Val Ala Asn His Met Gly Tyr Asp Gly Ala
        115                 120                 125

Gly Ser Ser Val Asp Tyr Ser Val Phe Lys Pro Phe Ser Ser Gln Asp
    130                 135                 140

Tyr Phe His Pro Phe Cys Phe Ile Gln Asn Tyr Glu Asp Gln Thr Gln
145                 150                 155                 160

Val Glu Asp Cys Trp Leu Gly Asp Asn Thr Val Ser Leu Pro Asp Leu
                165                 170                 175

Asp Thr Thr Lys Asp Val Val Lys Asn Glu Trp Tyr Asp Trp Val Gly
            180                 185                 190

Ser Leu Val Ser Asn Tyr Ser Ile Asp Gly Leu Arg Ile Asp Thr Val
        195                 200                 205

Lys His Val Gln Lys Asp Phe Trp Pro Gly Tyr Asn Lys Ala Ala Gly
    210                 215                 220

Val Tyr Cys Ile Gly Glu Val Leu Asp Gly Asp Pro Ala Tyr Thr Cys
225                 230                 235                 240
```

Pro Tyr Gln Asn Val Met Asp Gly Val Leu Asn Tyr Pro Ile Tyr Tyr
245 250 255

Pro Leu Leu Asn Ala Phe Lys Ser Thr Ser Gly Ser Met Asp Asp Leu
260 265 270

Tyr Asn Met Ile Asn Thr Val Lys Ser Asp Cys Pro Asp Ser Thr Leu
275 280 285

Leu Gly Thr Phe Val Glu Asn His Asp Asn Pro Arg Phe Ala Ser Tyr
290 295 300

Thr Asn Asp Ile Ala Leu Ala Lys Asn Val Ala Ala Phe Ile Ile Leu
305 310 315 320

Asn Asp Gly Ile Pro Ile Ile Tyr Ala Gly Gln Glu Gln His Tyr Ala
325 330 335

Gly Gly Asn Asp Pro Ala Asn Arg Glu Ala Thr Trp Leu Ser Gly Tyr
340 345 350

Pro Thr Asp Ser Glu Leu Tyr Lys Leu Ile Ala Ser Ala Asn Ala Ile
355 360 365

Arg Asn Tyr Ala Ile Ser Lys Asp Thr Gly Phe Val Thr Tyr Lys Asn
370 375 380

Trp Pro Ile Tyr Lys Asp Asp Thr Thr Ile Ala Met Arg Lys Gly Thr
385 390 395 400

Asp Gly Ser Gln Ile Val Thr Ile Leu Ser Asn Lys Gly Ala Ser Gly
405 410 415

Asp Ser Tyr Thr Leu Ser Leu Ser Gly Ala Gly Tyr Thr Ala Gly Gln
420 425 430

Gln Leu Thr Glu Val Ile Gly Cys Thr Thr Val Thr Val Gly Ser Asp
435 440 445

Gly Asn Val Pro Val Pro Met Ala Gly Gly Leu Pro Arg Val Leu Tyr
450 455 460

Pro Thr Glu Lys Leu Ala Gly Ser Lys Ile Cys Ser Ser Ser
465 470 475

**Description**

**Technical Field**

The present invention relates to the pharmaceutical use of proteases related to a protease derived from *Nocardiopsis sp.* NRRL 18262 (SEQ ID NO: 1), optionally in combination with a lipase and/or an amylase.

**Background Art**

Several commercial medicaments in the form of pancreatic enzyme supplements are known for the treatment of pancreatic exocrine insufficiency. The active ingredients of these products are digestive enzymes, mainly amylase, lipase and protease, which are normally produced in the pancreas and excreted to the upper part of the small intestine (the duodenum). The enzymes used in such medicaments derive from bovine or swine pancreas.

US 5614189 (EP 600868) describes the use of certain microbial lipases in pancreatic enzyme replacement therapy, for example in the treatment of patients suffering from cystic fibrosis.

WO 00/54799 describes the use of enzyme mixtures having lipolytic, proteolytic and amylolytic activity in the treatment of diabetes mellitus type I and II.

WO 02/060474 describes the use of certain lipases, proteases and amylases in the treatment of mal-digestion.

The protease derived from *Nocardiopsis sp.* NRRL 18262 (SEQ ID NO: 1), as well as its preparation and various industrial applications thereof are described in WO 88/03947 and WO 01/58276.

**Summary of the Invention**

The present invention relates to an acid stable protease of at least 90% identity to SEQ ID NO: 1, for use as a medicament for treatment of pancreatic exocrine insufficiency, optionally in combination with a lipase, and/or an amylase.

The invention also relates to the use of such proteases for the manufacture of a medicament for the treatment of PEI (pancreatic exocrine insufficiency), this use optionally further comprising the use of a lipase, and/or an amylase.

The invention furthermore relates to a pharmaceutical composition comprising such proteases, together with at least one pharmaceutically acceptable auxiliary material, optionally including a lipase and/or an amylase.

**Detailed Description of the Invention**

Enzymes

The term "protease" is defined herein as an enzyme that hydrolyses peptide bonds. It includes any enzyme belonging to the EC 3.4 enzyme group (including each of the thirteen subclasses thereof, these enzymes being in the following referred to as "belonging to the EC 3.4.-.- group"). The EC number refers to Enzyme Nomenclature 1992 from NC-IUBMB, Academic Press, San Diego, California, including supplements 1-5 published in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively. The nomenclature is regularly supplemented and updated; see e.g. the World Wide Web at hftp://www.chem.qmw.ac.uk/iubmb/enzyme/index.html.

Proteases are classified on the basis of their catalytic mechanism into the following groups: Serine proteases (S), Cysteine proteases (C), Aspartic proteases (A), Metallo proteases (M), and Unknown, or as yet unclassified, proteases (U), see Handbook of Proteolytic Enzymes, A.J.Barrett, N.D.Rawlings, J.F.Woessner (eds), Academic Press (1998), in particular the general introduction part.

The present invention relates to the pharmaceutical use of proteases of at least 70% identity to the protease of SEQ ID NO: 1, which is derived from *Nocardiopsis sp.* NRRL 18262, and described in WO 88/03947 and WO 01/58276.

Additional proteases of the invention are disclosed in WO 2004/111220, WO 2004/111221, WO 2004/111222, WO 2004/111223, WO 2005/035747, WO 2004/111219, hereby incorporated by reference.

Particular examples of proteases of the invention are derived from *Nocardiopsis dassonvillei* sub*sp. dassonvillei* DSM 43235 (SEQ ID NO: 2), *Nocardiopsis alba* DSM 15647 (SEQ ID NO: 3), *Nocardiopsis prasina* DSM 15648 (SEQ ID NO: 4), *Nocardiopsis prasina* DSM 15649 (SEQ ID NO: 5), as well as fragments, mutants, and variants thereof, such as Protease 22 (SEQ ID NO: 6). Optionally, each of SEQ ID NOs: 1-6 has a C-terminal extension consisting of one or more amino acids, for example non-polar or uncharged amino acids, such as one or more of Q, S, V, A, or P, preferably selected from the group consisting of: QSHVQSAP (SEQ ID NO:7), QSAP, QP, TL, TT, QL, TP, LP, TI, IQ, QP, PI, LT, TQ, IT, QQ, and PQ.

In particular embodiments, the proteases of the invention are selected from the group consisting of:

(a) proteases belonging to the EC 3.4.-.- enzyme group;
(b) serine proteases;
(c) serine proteases of peptidase family S2A;
(d) serine proteases of peptidase family S1E as described in Biochem.J. 290:205-218 (1993) and in MEROPS protease database, release 6.20, March 24, 2003, (www.merops.ac.uk). The database is described in Rawlings, N.D., O'Brien, E. A. & Barrett, A.J. (2002) MEROPS: the protease database. Nucleic Acids Res. 30, 343-346; and
(e) proteases derived from strains of *Nocardiopsis.*

For determining whether a given protease is a serine protease, and a family S2A protease, reference is made to the above Handbook and the principles indicated therein. Such determination can be carried out for all types of proteases, be it naturally occurring or wild-type proteases; or genetically engineered or synthetic proteases.

In particular embodiments, the degree of identity to SEQ ID NO: 1 is at least 90%, 91%, 92%, 93%, 94%, 95%, 96%, 97%, 98%, or at least 99%.

The protease of the invention is acid-stable, which means that the protease activity of the pure protease enzyme, in a dilution corresponding to $A_{280}$ = 1.0, and following incubation for 2 hours at 37°C in the following buffer: 100mM succinic acid, 100mM HEPES, 100mM CHES, 100mM CABS, 1mM $CaCl_2$, 150mM KCl, 0.01% Triton® X-100, pH 3.5; is at least 80% of the reference activity, as measured using the assay described in Example 2C of WO 01/58276 (substrate: Suc-AAPF-pNA, pH 9.0, 25°C). The term reference activity refers to the protease activity of the same protease, following incubation in pure form, in a dilution corresponding to $A_{280}$ = 1.0, for 2 hours at 5°C in the following buffer: 100mM succinic acid, 100mM HEPES, 100mM CHES, 100mM CABS, 1mM $CaCl_2$, 150mM KCl, 0.01% Triton® X-100, pH 9.0, wherein the activity is determined as described above. The term $A_{280}$= 1.0 means such concentration (dilution) of said pure protease which gives rise to an absorption of 1.0 at 280 nm in a 1cm path length cuvette relative to a buffer blank. The term pure protease refers to a sample with a $A_{280}/A_{260}$ ratio above or equal to 1.70 (see Example 2E of WO 01/58276), and which by a scan of a Coomassie stained SDS-PAGE gel is measured to have at least 95% of its scan intensity in the band corresponding to said protease (see Example 2A of WO 01/58276).

In still further particular embodiments, optionally, an additional protease may be used, for example a mammalian protease, for example in the form of pancreas extract from swine, or a microbial protease, for example derived from bacterial or fungal strains, such as *Bacillus, Pseudomonas, Aspergillus,* or *Rhizopus.* The protease may in particular be derived from a strain of *Aspergillus,* such as *Aspergillus oryzae* or *Aspergillus melleus,* in particular the product Prozyme 6™ (neutral, alkaline protease EC 3.4.21.63) which is commercially available from Amano Pharmaceuticals, Japan.

The protease of the invention may be used in combination with a <u>lipase</u>.

In the present context, a lipase means a carboxylic ester hydrolase EC 3.1.1.-, which includes activities such as EC 3.1.1.3 triacylglycerol lipase, EC 3.1.1.4 phospholipase A1, EC 3.1.1.5 lysophospholipase, EC 3.1.1.26 galactolipase, EC 3.1.1.32 phospholipase A1, EC 3.1.1.73 feruloyl esterase. In a particular embodiment, the lipase is an EC 3.1.1.3 triacylglycerol lipase.

In particular embodiments, the lipase is a mammalian lipase, for example in the form of pancreas extract from swine, or a microbial lipase, for example derived from bacterial or fungal strains, such as *Bacillus, Pseudomonas, Aspergillus,* or *Rhizopus.* The lipase may in particular be derived from a strain of *Rhizopus,* such as *Rhizopus javanicus, Rhizopus oryzae,* or *Rhizopus delemar,* for example the product Lipase D Amano 2000™ (also designated Lipase D2™) which is commercially available from Amano Pharmaceuticals, Japan.

In further particular embodiments, the lipase for use in the present invention is a recombinantly produced microbial lipase, for example derived from a fungus such as *Humicola* or *Rhizomucor,* from a yeast such as *Candida,* or from a bacterium such as *Pseudomonas.* In a preferred embodiment, the lipase is derived from a strain of *Humicola lanuginosa* or *Rhizomucor miehei.*

The *Humicola lanuginosa* (synonym *Thermomyces lanuginosus)* lipase (SEQ ID NO: 8) is described in EP 305216, and particular lipase variants are described in, for example, WO 92/05249, WO 92/19726, WO 94/25577, WO 95/22615, WO 97/04079, WO 97/07202, WO 99/42566, WO 00/32758, WO 00/60063, WO 01/83770, WO 02/055679, and WO 02/066622. Still further examples of fungal lipases are the cutinase from *Humicola insolens* which is described in EP 785994, and the phospholipase from *Fusarium oxysporum* which is described in EP 869167. Examples of yeast lipases are lipase A and B from *Candida antarctica* of which lipase A is described in EP 652945, and lipase B is described by, for example, Uppenberg et al in Structure, 2 (1994), 293. An example of a bacterial lipase is the lipase derived from *Pseudomonas cepacia,* which is described in EP 214761.

In a preferred embodiment, the lipase is at least 70% identical to the lipase of SEQ ID NO: 8. In additional preferred embodiments, the degree of identity to SEQ ID NO: 8 is at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%. In alternative embodiments, the degree of identity to SEQ ID NO: 8 is at least about 50%, 51%, 52%, 53%, 54%, 55%, 56%, 57%, 58%, 59%, 60%, 61%, 62%, 63%, 64%, 65%, 66%, 67%, 68%, or at least 69%.

In a still further preferred embodiment, the lipase, like the mammalian pancreatic lipase, is a 1,3-position specific lipase.

The protease of the invention, with or without a lipase as described above, may also be used in combination with an amylase.

In the present context, an amylase is an enzyme that catalyzes the endo-hydrolysis of starch and other linear and branched oligo- and polysaccharides. The amylose part of starch is rich in 1,4-alpha-glucosidic linkages, while the amylopectin part is more branched containing not only 1,4-alpha- but also 1,6-alpha-glucosidic linkages. In a particular embodiment, the amylase is an enzyme belonging to the EC 3.2.1.1 group.

In particular embodiments, the amylase is a mammalian amylase, for example in the form of pancreas extract from swine, or a microbial amylase, for example derived from bacterial or fungal strains, such as *Bacillus, Pseudomonas, Aspergillus,* or *Rhizopus.*

The amylase may in particular be derived from a strain of *Aspergillus,* such as *Aspergillus* niger, *Aspergillus oryzae* or *Aspergillus melleus,* for example either of the products Amylase A1™ derived from *Aspergillus oryzae* which is commercially available from Amano Pharmaceuticals, Japan, or Amylase EC™ derived from *Aspergillus melleus* which is commercially available from Extract-Chemie, Germany.

Other examples of fungal amylases are the *Aspergillus niger* amylase (SWISSPROT P56271), which is also described in Example 3 of WO 89/01969, and the *Aspergillus oryzae* amylase (SEQ ID NO: 9). Examples of variants of the *Aspergillus oryzae* amylase are described in WO 01/34784.

The alpha-amylase derived from *Bacillus licheniformis* is an example of a bacterial alpha-amylase. This amylase is, for example, described in WO 99/19467, together with other homologous bacterial alpha-amylases derived from, for example, *Bacillus amyloliquefaciens,* and *Bacillus stearothermophilus,* as well as variants thereof. Examples of additional amylase variants are those described in US patent no. 4,933,279; EP 722490, and EP 904360.

In a particular embodiment, the amylase is at least 70% identical to the amylase of SEQ ID NO: 9. In additional preferred embodiments, the degree of identity to SEQ ID NO: 9 is at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99%. In alternative embodiments, the degree of identity to SEQ ID NO: 9 is at least about 50%, 51%, 52%, 53%, 54%, 55%, 56%, 57%, 58%, 59%, 60%, 61%, 62%, 63%, 64%, 65%, 66%, 67%, 68%, or at least 69%.

Generally, the protease, lipase, and amylase enzymes (hereinafter "the enzyme(s)") for use according to the invention may be natural or wild-type enzymes obtained from animals, in particular mammals, for example human or swine enzymes; from plants, or from microorganisms, but also any mutants, variants, fragments etc. thereof exhibiting the desired enzyme activity, as well as synthetic enzymes, such as shuffled enzymes, and consensus enzymes.

In a specific embodiment, the enzyme(s) are low-allergenic variants, designed to invoke a reduced immunological response when exposed to animals, including man. The term immunological response is to be understood as any reaction by the immune system of an animal exposed to the enzyme(s). One type of immunological response is an allergic response leading to increased levels of IgE in the exposed animal. Low-allergenic variants may be prepared using techniques known in the art. For example the enzyme(s) may be conjugated with polymer moieties shielding portions or epitopes of the enzyme(s) involved in an immunological response. Conjugation with polymers may involve in vitro chemical coupling of polymer to the enzyme(s), e.g. as described in WO 96/17929, WO 98/30682, WO 98/35026, and/or WO 99/00489. Conjugation may in addition or alternatively thereto involve in vivo coupling of polymers to the enzyme(s). Such conjugation may be achieved by genetic engineering of the nucleotide sequence encoding the enzyme (s), inserting consensus sequences encoding additional glycosylation sites in the enzyme(s) and expressing the enzyme (s( in a host capable of glycosylating the enzyme(s), see e.g. WO 00/26354. Another way of providing low-allergenic variants is genetic engineering of the nucleotide sequence encoding the enzyme(s) so as to cause the enzymes to self-oligomerize, effecting that enzyme monomers may shield the epitopes of other enzyme monomers and thereby lowering the antigenicity of the oligomers. Such products and their preparation is described e.g. in WO 96/16177. Epitopes involved in an immunological response may be identified by various methods such as the phage display method described in WO 00/26230 and WO 01/83559, or the random approach described in EP 561907. Once an epitope has been identified, its amino acid sequence may be altered to produce altered immunological properties of the enzyme(s) by known gene manipulation techniques such as site directed mutagenesis (see e.g. WO 00/26230, WO 00/26354 and/or WO 00/22103) and/or conjugation of a polymer may be done in sufficient proximity to the epitope for the polymer to shield the epitope.

In particular embodiments, the protease, lipase, and/or amylase enzymes are (i) stable at pH 4-8, preferably also at pH 3-4, more preferably at pH 3.5; (ii) active at pH 4-9, preferably 4-8, more preferably at pH 6.5; (iii) stable against degradation by pepsin and other digestive proteases (such as pancreas proteases, i.e., mainly trypsin and chymotrypsin); and/or (iv) stable and/or active in the presence of bile salts

The term "in combination with" refers to the combined use according to the invention of the protease, lipase, and/or amylase. The combined use can be simultaneous, overlapping, or sequential, these three terms being generally interpreted in the light of the prescription made by the physician.

The term "simultaneous" refers to circumstances under which the enzymes are active at the same time, for example when they are administered at the same time as one or more separate pharmaceutical products, or if they are administered in one and the same pharmaceutical composition.

The term "sequential" refers to such instances where one and/or two of the enzymes are acting first, and the second and/or third enzyme subsequently. A sequential action can be obtained by administering the enzymes in question as separate pharmaceutical formulations with desired intervals, or as one pharmaceutical composition in which the enzymes in question are differently formulated (compartmentalized), for example with a view to obtaining a different release time, providing an improved product stability, or to optimizing the enzyme dosage.

The term "overlapping" refers to such instances where the enzyme activity periods are neither completely simultaneous nor completely sequential, viz. there is a certain period in which the enzymes are both, or all, active.

The term "a", for example when used in the context of the protease, lipase, and/or amylase of the invention, means at least one. In particular embodiments, "a" means "one or more," or "at least one", which again means one, two, three, four, five etc.

For purposes of the present invention the degree of identity between two amino acid sequences is determined by the program "align" which is a Needleman-Wunsch alignment (i.e. a global alignment). The sequences are aligned by the program, using the default scoring matrix BLOSUM50 is used. The penalty for the first residue of a gap is 12, and for further residues of a gap the penalties are 2.

"Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," Methods in Enzymology 183:63- 98). FASTA protein alignments use the Smith-Waterman algorithm with no limitation on gap size (see "Smith-Waterman algorithm", T. F. Smith and M. S. Waterman (1981) J. Mol. Biol. 147:195-197).

The activity of the enzyme(s) of the invention can be measured using any suitable assay. Generally, assay-pH and assay-temperature are to be adapted to the enzyme in question. Examples of assay-pH-values are pH 2, 3, 4, 5, 6, 7, 8, 9, 10, 11, or 12. Examples of assay-temperatures are 30, 35, 37, 40, 45, 50, 55, 60, 65, 70, 80, 90, or 95°C.

For example, protease activity can be measured using any assay, in which a substrate is employed, that includes peptide bonds relevant for the specificity of the protease in question.

Examples of suitable enzyme assays are included in the experimental part. Other examples are the Ph.Eur. assays for lipase and amylase activity.

Medicament

In the present context, the term "medicament" means a compound, or mixture of compounds, that treats, prevents and/or alleviates the symptoms of disease. The medicament may be prescribed by a physician, or it may be an over-the-counter product.

Pharmaceutical Compositions

Isolation, purification, and concentration of the enzyme(s) of the invention may be carried out by conventional means.

In a particular embodiment, concentrated solid or liquid preparations of each of the enzyme(s) are prepared separately. These concentrates may also, at least in part, be separately formulated, as explained in more detail below.

In a further particular embodiment, the enzyme(s) are incorporated in the pharmaceutical compositions of the invention in the form of solid concentrates. The enzyme(s) can be brought into the solid state by various methods as is known in the art. For example, the solid state can be either crystalline, where the enzyme molecules are arranged in a highly ordered form, or a precipitate, where the enzyme molecules are arranged in a less ordered, or disordered, form.

Crystallization may, for example, be carried out at a pH close to the pl of the enzyme(s) and at low conductivity, for example 10 mS/cm or less, as described in EP 691982 (see also Example 2 herein).

Various precipitation methods are known in the art, including precipitation with salts, such as ammonium sulphate, and/or sodium sulphate; with organic solvents, such as ethanol, and/or isopropanol; or with polymers, such as PEG (Poly Ethylene Glycol). In the alternative, the enzyme(s) can be precipitated from a solution by removing the solvent (typically water) by various methods known in the art, e.g. lyophilization, evaporation (for example at reduced pressure), and/or spray drying.

In a further particular embodiment, the solid concentrate of the enzyme(s) has a content of active enzyme protein of at least 50% (w/w) by reference to the total protein content of the solid concentrate. In still further particular embodiments, the content of active enzyme protein, relative to the total protein content of the solid concentrate is at least 55, 60, 65, 70, 75, 80, 85, 90, or at least 95% (w/w). The protein content can be measured as is known in the art, for example using a commercial kit, such as Protein Assay ESL, order no. 1767003, which is commercially available from Roche, or on the basis of the method described in Example 8 of WO 01/58276.

A pharmaceutical composition of the invention comprises the enzyme(s), preferably in the form of concentrated enzyme preparations, more preferably solid concentrates, together with at least one pharmaceutically acceptable auxiliary, or subsidiary, material such as (i) at least one carrier and/or excipient; or (ii) at least one carrier, excipient, diluent, and/or adjuvant. Non-limiting examples of, optional, other ingredients, all pharmaceutically acceptable, are disintegrators, lubricants, buffering agents, moisturizing agents, preservatives, flavouring agents, solvents, solubilizing agents, suspending agents, emulsifiers, stabilizers, propellants, and vehicles.

Generally, depending i.a. on the medical indication in question, the composition of the invention may be designed for all manners of administration known in the art, including enteral administration (through the alimentary canal), and parenteral administration, for example by injection (such as subcutaneous, intramuscular, or intravenous, etc.). Thus, the composition may be in solid, semi-solid, liquid, or gaseous form, such as tablets, capsules, powders, granules, microspheres, ointments, creams, foams, solutions, suppositories, injections, inhalants, gels, microspheres, lotions, and aerosols.

The following methods and auxiliary materials are merely exemplary and are in no way limiting.

For solid oral preparations, the enzyme(s) can be used alone or in combination with appropriate additives to make tablets, powders, granules or capsules, for example, with conventional carriers, such as lactose, mannitol, corn starch, or potato starch; with excipients or binders, such as crystalline, or microcrystalline, cellulose, cellulose derivatives, acacia, corn starch, or gelatins; with disintegrators, such as corn starch, potato starch, or sodium carboxymethylcellulose; with lubricants, such as carnauba wax, white wax, shellac, waterless colloid silica, macrogol 6000, povidone, talc, monolein, or magnesium stearate; and if desired, with diluents, adjuvants, buffering agents, moistening agents, preservatives such as methylparahydroxybenzoate (E218), colouring agents such as titanium dioxide (E171), and flavouring agents such as saccharose, saccharin, orange oil, lemon oil, and vanillin. Oral preparations are examples of preferred preparations for treatment of the medical indication of PEI.

The enzyme(s) can also, quite generally, be formulated into preparations for injection, or into liquid oral preparations, by dissolving, suspending, or emulsifying them in an aqueous solvent such as water, or in non-aqueous solvents such as vegetable or other similar oils, synthetic aliphatic acid glycerides, esters of higher aliphatic acids, propylene glycol, polyethylene glycol such as PEG 4000, or lower alcohols such as linear or ramified C1-C4 alcohols, for example 2-propanol; and if desired, with conventional subsidiary materials or additives such as solubilizers, adjuvants, diluents, isotonic agents, suspending agents, emulsifying agents, stabilizers, and preservatives.

The enzyme(s) can furthermore, still quite generally, be utilized in aerosol formulation to be administered via inhalation, for example by formulation into pressurized acceptable propellants such as dichlorodifluoromethane, propane, nitrogen, and the like.

Furthermore, the enzyme(s) can generally be made into suppositories for rectal administration by mixing with a variety of bases such as emulsifying bases or water-soluble bases. The suppository can include vehicles such as cocoa butter, carbowaxes and polyethylene glycols, which melt at body temperature, yet are solidified at room temperature.

The use of liposomes as a delivery vehicle is another method of possible general interest. The liposomes fuse with the cells of the target site and deliver the contents of the lumen intracellularly. The liposomes are maintained in contact with the cells for sufficient time for fusion, using various means to maintain contact, such as isolation, binding agents, and the like. In one aspect of the invention, liposomes are designed to be aerosolized for pulmonary administration. Liposomes may be prepared with purified proteins or peptides that mediate fusion of membranes, such as Sendai virus or influenza virus, etc. The lipids may be any useful combination of known liposome forming lipids, including cationic or zwitterionic lipids, such as phosphatidylcholine. The remaining lipid will normally be neutral or acidic lipids, such as cholesterol, phosphatidyl serine, phosphatidyl glycerol, and the like. For preparing the liposomes, the procedure described by Kato et al. (1991) J. Biol. Chem. 266:3361 may be used.

Unit dosage forms for oral or rectal administration such as syrups, elixirs, powders, and suspensions may be provided wherein each dosage unit, for example, teaspoonful, tablespoonful, capsule, tablet or suppository, contains a predetermined amount of the enzyme(s). Similarly, unit dosage forms for injection or intravenous administration may comprise the enzyme(s) in a composition as a solution in sterile water, normal saline, or another pharmaceutically acceptable carrier.

The term "unit dosage form", as used herein, refers to physically discrete units suitable as unitary dosages for human and animal subjects, each unit containing a predetermined quantity of enzyme(s) in an amount sufficient to produce the desired effect.

In a particular embodiment, the pharmaceutical composition of the invention is for enteral, preferably oral, administration.

In further particular embodiments, the oral composition is (i) a liquid composition containing crystals of the enzyme(s); (ii) a liquid suspension of sediments of (highly) purified enzyme(s); (iii) a gel containing the enzyme(s) in solid or solubilized form; (iv) a liquid suspension of immobilized enzyme(s) or of enzymes adsorbed to particles and the like; or (v) a solid composition in the form of enzyme(s)-containing powder, pellets, granules, or microspheres, if desired in the form of tablets, capsules, or the like, that are optionally coated, for example with an acid-stable coating.

In another particular embodiment of the composition, the enzyme(s) are compartmentalized, viz. separated from each other, for example by means of separate coatings.

In a still further particular embodiment of the composition, the protease is separated from other enzyme components of the composition, such as the lipase, and/or the amylase.

The dosage of the enzyme(s) will vary widely, depending on the specific enzyme(s) to be administered, the frequency of administration, the manner of administration, the severity of the symptoms, and the susceptibility of the subject to side effects, and the like. Some of the specific enzymes may be more potent than others.

The amide (peptide) bonds, as well as the amino and carboxy termini, may be modified for greater stability on oral administration. For example, the carboxy terminus may be amidated.

Methods of Treatment

The protease of the invention, optionally in combination with a lipase, and/or an amylase (the enzyme(s) of the invention), is useful in the therapeutic, and/or prophylactic, treatment of various diseases or disorders in animals. The term "animal" includes all animals, and in particular human beings. Examples of animals are non-ruminants, and ruminants, such as sheep, goats, horses, and cattle, e.g. beef cattle, cows, and young calves. In a particular embodiment, the animal is a non-ruminant animal. Non-ruminant animals include mono-gastric animals, e.g. pigs or swine (including, but not limited to, piglets, growing pigs, and sows); poultry such as turkeys, ducks and chicken (including but not limited to broiler chicks, layers); young calves; pets such as cat, and dog; and fish (including but not limited to salmon, trout, tilapia, catfish and carps; and crustaceans (including but not limited to shrimps and prawns). In a particular embodiment the animal is a mammal, more in particular a human being.

For example, the enzyme(s) are useful in the treatment of digestive disorders like mal-digestion or dyspepsia that are often caused by a deficient production and/or secretion into the gastrointestinal tract of digestive enzymes normally secreted from, i.a., the stomach, and the pancreas.

Further, the enzyme(s) are particularly useful in the treatment of PEI. PEI can be verified using, i.a., the Borgström test (JOP. J Pancreas (Online) 2002; 3(5):116-125), and it may be caused by diseases and conditions such as pancreatic cancer, pancreatic and/or gastric surgery, e.g. total or partial resection of the pancreas, gastrectomy, post gastrointestinal bypass surgery (e.g. Billroth II gastroenterostomy); chronic pancreatitis; Shwachman Diamond Syndrome; ductal obstruction of the pancreas or common bile duct (e.g. from neoplasm); and/or cystic fibrosis (an inherited disease in which a thick mucus blocks the ducts of the pancreas). The enzyme(s) may also be useful in the treatment of acute pancreatitis.

The effect of the enzyme(s) on digestive disorders can be measured as generally described in EP 0600868, in which Example 2 describes an *in vitro* digestibility test for measuring lipase stability test under gastric conditions, and Example 3 an *in vitro* digestibility test for lipase activity in the presence of bile salts. Corresponding tests can be set up for the protease and amylase. Also WO 02/060474 discloses suitable tests, for example (1) an *in vitro* test for measuring lipid digestion in a swine test feed, and (2) an in vivo trial with pancreas insufficient swine in which the digestibility of fat, protein and starch is measured.

In a particular embodiment, the effect of the protease of the invention is measured using the *in vitro* pancreas insufficiency digestion model of Example 1 herein, in which various other substrates may be used as desired, for example animal protein, other vegetable proteins, cereals, animal or vegetable fats and oils, as well as any mixtures thereof.

In other particular embodiments, the effect of the protease of the invention is measured using the *in vivo* screening test for protease efficacy of Example 4, or the full *in vivo* digestibility trial of Example 5.

As another example, the enzyme(s) are useful in the treatment of Diabetes mellitus type I, and/or type II, in particular for adjuvant treatment in a diabetes therapy of digestive disorders usually accompanying this disease, with a view to diminishing late complications.

The effect on Diabetes mellitus of the enzyme(s) may be determined by one or more of the methods described in WO 00/54799, for example by controlling the level of glycosylated haemoglobin, the blood glucose level, hypoglycaemic attacks, the status of fat-soluble vitamins like vitamins A, D and E, the required daily dosage of insulin, the body-weight index, and hyper glycaemic periods.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

Various references are cited herein, the disclosures of which are incorporated by reference in their entireties.

**Examples**

**Example 1: *In vitro* Pancreatic Insufficiency Digestion Model**

A purified preparation of the protease derived from *Nocardiopsis sp.* NRRL 18262 (SEQ ID NO: 1) was prepared as generally described in Example 2 of WO 01/58276, and tested in an *in vitro* model simulating the digestion in individuals suffering from pancreatic insufficiency.

The *in vitro* system consists of 24 flasks in which a substrate (based on maize and soybean meal (SBM)) was initially incubated with HCl/pepsin (simulating gastric digestion), and subsequently with two reduced levels of pancreatin, simulating intestinal digestion in an individual with partial and complete pancreatic insufficiency. A positive control experiment was also included with a normal level of pancreatin.

10 of the flasks were dosed with the protease at the start of the gastric phase whereas the remaining flasks served as blanks. At the end of the intestinal incubation phase samples of *in vitro* digesta were removed and analysed for solubilised and digested protein.

Outline of *in vitro* digestion procedure

| Components added | pH | Temperature | Time course | Simulated digestion phase |
|---|---|---|---|---|
| 10 g maize/-SBM substrate (6:4), 41 ml HCl (0.105M) | 3.0 | 40°C | t=0 min | Mixing |
| 5 ml HCl (0.105M) / pepsin (3000 U/g substrate), 100 mg protease EP/kg substrate) | 3.0 | 40°C | t=30 min | Gastric digestion |
| 16 ml H$_2$O | 3.0 | 40°C | t= 1.0 hour | Gastric digestion |
| 7 ml NaOH (0,39M) | 6.8 | 40°C | t=1.5 hours | Intestinal digestion |
| 5 ml NaHCO$_3$ (1 M) / pancreatin (0, 4 or 8 mg/g substrate) | 6.8 | 40°C | t=2.0 hours | Pancreatin-defective intestinal digestion (plus a positive control) |
| Terminate incubation | 7.0 | 40°C | t=6.0 hours | |

Conditions

Substrate:     4 g SBM, 6 g maize (premixed)
HCl:           0.105 M for 1.5 hours (i.e. 30 min HCl-substrate premixing)
pepsin:        Sigma P-7000; 3000 U /g substrate for 1 hour
pancreatin:    Sigma P-7545; 0, 4, or 8 mg / g substrate for 4 hours (the assumed normal level of pancreatin being 8 mg/g)
protease:      100 mg protease enzyme protein (EP) / kg of substrate (Enzyme Protein was calculated on the basis of the A$_{280}$ values and the amino acid sequences (amino acid compositions) using the principles outlined in S.C.Gill & P.H. von Hippel, Analytical Biochemistry 182, 319-326, (1989))
pH:            3.0 stomach step / 6.8-7.0 intestinal step
temperature:   40°C
Replicates:    5 (4)

Solutions

0.39 M NaOH
0.105 M HCl
0.105 M HCl containing 6000 U pepsin per 5 ml

1 M NaHCO$_3$ containing 16 mg pancreatin per ml
125 mM NaAc-buffer, pH 6.0

Experimental procedure for *in vitro* model

The experimental procedure was according to the above outline. pH was measured at time 1, 2.5, and 5.5 hours. Incubations were terminated after 6 hours and samples of 30 ml were removed and placed on ice before centrifugation (10000 x g, 10 min, 4°C). Supernatants were removed and stored at -20°C.

Analysis

All samples were analysed for content of solubilised and digested protein using gel filtration.

Estimation of solubilised and digested protein

The content of solubilised protein in supernatants from *in vitro* digested samples was estimated by quantifying crude protein (CP) using gel filtration HPLC. Supernatants were thawed, filtered through 0.45 $\mu$m polycarbonate filters and diluted (1:50, v/v) with H$_2$O. Diluted samples were chromatographed by HPLC using a Superdex Peptide PE (7.5 x 300 mm) gel filtration column (Global). The eluent used for isocratic elution was 50 mM sodium phosphate buffer (pH 7.0) containing 150 mM NaCl. The total volume of eluent per run was 26 ml and the flow rate was 0.4 ml/min. Elution profiles were recorded at 214 nm and the total area under the profiles was determined by integration. To estimate protein content from integrated areas, a calibration curve (R$^2$=0.9993) was made from a dilution series of an *in vitro* digested reference maize/-SBM sample with known total protein content. The protein determination in this reference sample was carried out using a standard method (in this case the Kjeldahl method for determination of % nitrogen; A.O.A.C. (1984) Official Methods of Analysis 14th ed., Washington DC).

The content of digested protein was estimated by integrating the chromatogram area corresponding to peptides and amino acids having a molecular mass of 1500 Dalton or below (Savoie,L.; Gauthier,S.F. Dialysis Cell For The In-vitro Measurement Of Protein Digestibility. J. Food Sci. 1986, 51, 494-498; Sabinszky,L.; Van,D.M.J.M.; Boer,H.; Den, H.L.A. An In-vitro Method for Prediction of The Digestible Crude Protein Content in Pig Feeds. J. Sci. Food Agr. 1990, 50, 173-178; Boisen,S.; Eggum,B.O. Critical Evaluation of In-vitro Methods for Estimating Digestibility in Simple-Stomach Animals. Nutrition Research Reviews 1991, 4, 141-162). To determine the 1500 Dalton dividing line, the gel filtration column was calibrated using cytochrome C (Boehringer, Germany), aprotinin, gastrin I, and substance P (Sigma Aldrich, USA), as molecular mass standards.

The experimental results are shown in Table 1 and also visualized in Fig. 1. In Table 1, the column "Enzyme" shows the amount of pancreatin (abbreviated "pan") per gram of substrate, as well as the amount of the *Nocardiopsis* protease (in square brackets). In the next column, "n" is the number of replicates of each experiment. The following cluster of columns shows the percentage of digestible Crude Protein (abbreviated %dig.CP), including the Standard Deviation (SD), and the significance superscript letter as explained in the footnote below the table. And finally, the last cluster of columns shows the percentage of soluble Crude Protein (abbreviated %sol.CP), also including SD and significance superscript letters.

As it appears from Table 1, there is a strong tendency (P<0.10) for the addition of the *Nocardiopsis* protease to improve the percentage of solubilized, as well as digestible protein, and this is so in the experiment with 4 mg/g pancreatin, as well as in the experiment with 0 mg/g pancreatin (compare "4 mg pan, [100]" with "4 mg pan, [0];" and "0 mg pan, [100]" with "0 mg pan, [0]"). This means that the *Nocardiopsis* protease is able to compensate for the partial or complete absence of pancreatin.

Table 1

| Enzyme [mg EP/kg] | N | Of total protein %dig. CP | | SD | %sol.CP | | SD |
|---|---|---|---|---|---|---|---|
| 8 mg pan, [0] | 5 | 54.7 | C | 3.0 | 98.2 | C | 4.0 |
| 4 mg pan, [0] | 5 | 47.0 | A | 2.2 | 88.1 | B | 4.0 |
| 0 mg pan, [0] | 4 | 45.1 | A | 4.1 | 83.8 | A | 2.4 |
| 4 mg pan, [100] | 5 | 55.0 | C | 1.2 | 95.8 | C | 1.8 |

(continued)

| Enzyme [mg EP/kg] | N | Of total protein %dig. CP | | SD | %sol.CP | | SD |
|---|---|---|---|---|---|---|---|
| 0 mg pan, [100] | 5 | 49.9 | B | 0.7 | 88.8 | B | 1.2 |

LSD 90%
Values within a column with different capital superscript letters indicate a strong tendency for a difference (1-way ANOVA, Least Significant Difference (LSD) test, P<0.10). SD = Standard Deviation.

**Example 2: Preparation of Crystallized Protease Preparations**

The protease of SEQ ID NO: 1 was fermented as described in Example 1, and the protease-containing broth was harvested on a centrifuge at pH 4.5. The resulting supernatant was subjected to ultra-filtration using a membrane with a cut-off value of 6 kDal, and to diafiltration until a conductivity of 2 mS/cm in the protease-containing solution. The content of protease was approximately 100 mg/mL.

The concentrated and diafiltered protease solution is crystallized by adjusting pH with sodium hydroxide to pH 8.5, i.e. close to the pl of the protease (which is 9.3). After pH adjustment the solution is left over night at room temperature, and crystallization takes place.

The following day the crystallized protease is harvested by centrifugation.

**Example 3: Enzyme Assays**

Protease

Substrate: Suc-AAPF-pNA (Sigma® S-7388).
Assay buffer: 100mM succinic acid, 100mM HEPES, 100mM CHES, 100mM CABS, 1 mM $CaCl_2$, 150mM KCI, 0.01 % Triton® X-100 adjusted to pH 9.0 with HCl or NaOH.
Assay temperature: 25°C.

300$\mu$l diluted protease sample was mixed with 1.5ml of the assay buffer and the activity reaction was started by adding 1.5ml pNA substrate (50mg dissolved in 1.0ml DMSO and further diluted 45x with 0.01% Triton® X-100) and, after mixing, the increase in $A_{405}$ was monitored by a spectrophotometer as a measurement of the protease activity. The protease samples were diluted prior to the activity measurement in order to ensure that all activity measurements fell within the linear part of the dose-response curve for the assay.

Protease FIP assay

Protease activity may also be determined using the FIP assay (Federation Internationale Pharmaceutique), 1 FIP-unit = 1 Ph.Eur.-unit (European Pharmacopoeia). This assay is described, together with other FIP assays in: Federation Internationale Pharmaceutique, Scientific Section: International Commission for the standardisation of pharmaceutical enzymes. a) "Pharmaceutical Enzymes," Editors: R. Ruyssen and A. Lauwers, E. Story Scientia, Ghent, Belgium (1978), b) European Pharmacopoeia. See also Deemester et al in Lauwers A, Scharpé S (eds): Pharmaceutical Enzymes, New York, Marcel Dekker, 1997, p. 343-385.

Principle: The substrate casein is hydrolysed by protease at pH 7.5 and at a temperature of 35°C. The reaction is stopped by addition of trichloroacetic acid, and non-degraded casein is filtered off. The quantity of peptides remaining in solution is determined by spectrophotometry at 275 nm.

Definition of the activity: The protease activity is determined as the quantity of peptides not precipitated by a 5.0% (wt/vol, i.e. 5.0 g/100ml) solution of trichloroacetic acid, by reference to a pancreas reference powder (protease reference standard) of known FIP activity.

Materials and methods:

Casein solution:

1.25 g casein (dry matter), e.g. Calbiochem no. 218680, is suspended in water until a practically clear solution is obtained. pH is adjusted to 8.0, and the solution is diluted with water to a final volume of 100 ml. Here and in the following, water means deionized water.

Borate buffer pH 7.5:

2.5 g sodium chloride, 2.85 g disodium tetraborate and 10.5 g boric acid are dissolved in 900 ml water, pH is adjusted to pH 7.5+/-0.1 and diluted to 1000 ml with water.

Filter paper:

Folded filters with a diameter of 125 mm, e.g. Schleicher & Schuell no. 1573½. Test of filter paper: Filter 5 ml of 5.0 % trichloro acetic acid through the filter. The absorption at 275 nm of the filtrate should be less than 0.04, using unfiltered trichloroacetic acid solution as a blank.

Protease reference standard:

Protease (pancreas) commercially available from the International Commission on Pharmaceutical Enzymes, Centre for Standards, Harelbekestraat 72, B-9000 Ghent, Belgium. The standard has a labelled activity (A) in FIP/Ph.Eur.-units/g. Accurately weigh a quantity corresponding to approx. 130 protease-FIP/Ph.Eur.-units. Add a spatula tip of sea sand, wet with a few drops of ice-cold 0.02M calcium chloride (pH 6.0-6.2), and triturate the whole with a flat-ended glass rod. Dilute with approx. 90 ml of the same ice-cold calcium chloride solution and stir the suspension for 15 to 30 minutes in an ice-bath. pH is adjusted to 6.1 and the volume is adjusted to 100 ml with the same calcium chloride solution. 5.0 ml of this suspension is diluted with borate buffer pH 7.5 to 100 ml. For the activity test, 1.0, 2.0 and 3.0 ml of this solution is used as reference (in what follows designated S1, S2, and S3, S for Standard).

Test suspension:

Prepare a suspension of the sample as described above for the protease reference standard, using a sample amount equivalent to approx. 260 FIP/Ph.Eur.-units. pH is adjusted to 6.1 and water is added to 100 ml. 5.0 ml of this solution is mixed with 5 ml of calcium chloride solution. 5 ml of this dilution is further diluted to 100 ml with borate buffer. Use 2.0 ml of this solution for the assay (in what follows the sample is designated Un, sample of unknown activity, number n).

Assay procedure (activity test):

The assay is performed for the three reference suspensions (S1, S2, S3) and for the sample suspension (Un), all in triplicate. One blank per sample is sufficient (designated S1 b, S2b, S3b, and Unb, respectively). A blind (B) is prepared without without sample/standard as compensation liquid for the spectrophotometer. Borate buffer is added to tubes as follows: Blind (B) 3.0 ml; sample (Un) 1.0 ml; standards (S1, S2 and S3) 2.0, 1.0 and 0 ml, respectively. Protease reference standard is added to S1, S2 and S3 as follows: 1.0, 2.0, and 3.0 ml, respectively. The test suspension is added to the sample tubes as follows (Un): 2.0 ml. 5 ml trichloro acetic acid is added to all blinds (S1b, S2b, S3b, Unb and B) followed by immediate mixing. All tubes are stopped with a glass stopper and placed together with the substrate solution in a water-bath at constant temperature (35+/- 0.5°C). When temperature equilibration is reached, at time zero, 2.0 ml casein solution is added to tubes S1, S2, S3 and Un, followed by immediate mixing. Exactly 30 minutes after, 5.0 ml. trichloro acetic acid is added to each of tubes S1, S2, S3 and Un, followed by immediate mixing. The tubes are withdrawn from the water bath and allowed to stand at room temperature for 20 minutes to complete the precipitation of the proteins. The content of each tube is filtered twice through the same filter, and the absorption of the filtrates is measured at 275 nm using the filtrate from tube B as compensation liquid. The activity of the sample (Un) in FIP units is calculated relative to the known labelled activity (A) of the standards (S1, S2, S3). The absorption values minus the respective blinds (e.g. the absorption of S1 minus the absorption of S1b) should lie in the interval of 0.15 - 0.60.

Lipase

Substrate: para-Nitro-Phenyl (pNP) Valerate
Assay pH: 7.7
Assay temperature: 40°C
Reaction time: 25 min

The digested product with yellow colour has a characteristic absorbance at 405nm. Its quantity is determined by spectrophotometry. One lipase unit is the amount of enzyme which releases 1 micromole titratable butyric acid per minute under the given assay conditions. A more detailed assay description, AF95/6-GB, is available on request from Novozymes A/S, Krogshoejvej 36, DK-2880 Bagsvaerd, Denmark.

Amylase

Substrate: Phadebas tablets (Pharmacia Diagnostics; cross-linked, insoluble, blue-coloured starch polymer, which is mixed with bovine serum albumin and a buffer substance, and manufactured into tablets)
Assay Temperature: 37°C
Assay pH: 4.3
Reaction time: 20 min

After suspension in water the starch is hydrolyzed by the alpha-amylase, giving soluble blue fragments. The absorbance of the resulting blue solution, measured at 620 nm, is a function of the alpha-amylase activity. One Fungal alpha-Amylase Unit (1 FAU) is the amount of enzyme which breaks down 5.26 g starch (Merck, Amylum solubile Erg. B. 6, Batch 9947275) per hour at the standard assay conditions. A more detailed assay description, APTSMYQI-3207, is available on request from Novozymes A/S, Krogshoejvej 36, DK-2880 Bagsvaerd, Denmark.

**Example 4: *In vivo* screening test for protease efficacy**

The protease described in Example 1 was tested in female Göttingen minipigs (Ellegaard). In the minipigs, the pancreatic duct was ligated to induce Pancreatic Exocrine Insufficiency (PEI), and they were fitted with an ileo-caecal re-entrant cannula, all under halothane anaesthesia and at a weight of about 25 kg, as described in Tabeling et al., J. 1999, Studies on nutrient digestibilities (pre-caecal and total) in pancreatic duct-ligated pigs and the effects of enzyme substitution, J. Anim. Physiol. A. Anim. Nutr. 82: 251-263 (hereinafter referred to as "Tabeling 1999"); and in Gregory et al., J. 1999. Growth and digestion in pancreatic duct ligated pigs, Effect of enzyme supplementation in "Biology of the Pancreas in Growing Animals" (SG Pierzynowski & R. Zabielski eds), Elsevier Science BV, Amsterdam, pp 381-393 (hereinafter referred to as "Gregory et al 1999"). A period of at least 4 weeks was allowed for recovery from surgery, before studies were commenced. Prior to study begin, the PEI status of each pig was confirmed via the stool chymotrypsin test (commercially available from Immundiagnostik AG, Wiesenstrasse 4, D-64625 Bensheim, Germany, with catalogue No. K 6990).

During the studies, the pigs were housed in modified metabolism cages on a 12:12h light-dark cycle and allowed free access to water and fed two meals/day. To assess protease efficacy, the pigs were fed a 250 g test meal mixed with 1 liter of water, 0.625 g $Cr_2O_3$ (chromic oxide marker) and into which differing amounts of protease (0, 1000, 2500, 6000 FIP U protease/meal (protease FIP units, see Example 3)) were mixed immediately before feeding. The test meal contained 21.4% protein, 51.9% starch, 2.6% fat, and had the following composition (g/100g dry matter): Fish meal 3.5, poultry meat meal 10.2, wheat flour 29.5, shelled rice 14, potato starch 11, maize starch 14, casein 5.9, cellulose powder 4.3, vitamins, minerals and trace elements 7.6 (as per the nutritional requirement for pigs/piglets, see e.g. Table A of WO 01/58276).

Ileal chyme was collected on ice for a total of 8h after first appearance of the meal marker in the ileum (green chyme) and stored at -20°C before analysis. At least one day washout was allowed between separate determinations.

In brief, the frozen samples were freeze-dried and analysed for dry matter (DM) and crude protein. DM was estimated by weight after freeze-drying followed by 8h incubation at 103°C. Crude protein was calculated as nitrogen (N) multiplied by a factor 6.25, i.e. Crude protein (g/kg)= N (g/kg) x 6.25 as stated in Animal Nutrition, 4th edition, Chapter 13 (Eds. P. McDonald, R. A. Edwards and J. F. D. Greenhalgh, Longman Scientific and Technical, 1988, ISBN 0-582-40903-9). The nitrogen content was determined by the Kjeldahl method (Naumann and Bassler, 1993, Die chemische Untersuchung von Futtermitteln. 3 edition VDLUFA-Verlag, Darmstadt, Germany (VDLUFA = Verband Deutscher Landwirtschaftlicher Untersuchungs- und Forschungsanstalten).

Calculation of apparent pre-caecal protein digestibility was made according to the formula:

$$\text{Apparent digestibility (\%)} = 100 - \left[ \frac{\% \ Cr_2O_3 \ \text{in feed}}{\% \ Cr_2O_3 \ \text{in sample}} \cdot \frac{\% \ \text{protein in sample}}{\% \ \text{protein in feed}} \cdot 100 \right]$$

in which $Cr_2O_3$ and protein were expressed as g/100g dry matter.

Table 2: Influence of enzyme supplementation on apparent protein digestibility

| Enzyme Supplement | 0 | 1000 FIP U | 2500 FIPU | 6000 FIP U |
|---|---|---|---|---|
| No supplement | 14.7 ± 2.1 | | | |
| Pancreatin | | 31.7 ± 12.4 | 59.4 ± 4.9 | 70.7 ±0.9 |
| Protease | | 55.2 ± 4.3 | 61.7 ± 4.8 | 68.4 ± 3.9 |
| Values are mean ± SD | | | | |

## Example 5: Full *in vivo* digestibility trial

The protease described in Example 1 was tested in female Göttingen minipigs (Ellegaard) in which the pancreatic duct was ligated to induce PEI, and they were fitted with an ileo-caecal re-entrant cannula, all under halothane anaesthesia and at a weight of about 25 kg, as previously described (Tabeling 1999; Gregory et al 1999). Control minipigs were prepared in similar manner, but the pancreatic duct was left intact. A period of at least 4 weeks was allowed, for recovery from surgery, before studies were commenced. Prior to study begin, the PEI status of each pig was confirmed via the stool chymotrypsin test (see Example 4).

The pigs were allowed free access to water and fed two 250 g meals/day, at 08.00 and 20.00h, of a finely milled diet (as in Example 4), mixed with 1 litre water, 0.625 g $Cr_2O_3$ and into which differing amounts of protease (0, 6000 FIP U protease/meal) were mixed immediately before feeding. Each dose was fed to the pigs for 2 weeks and ileal chyme was collected on ice for 12h for the final 3 days. The samples were stored at -20°C until analysis.

In brief, the frozen samples were freeze-dried and analysed for dry matter (DM) and crude protein. DM and crude protein was estimated and pre-caecal protein digestibility (apparent digestibility) calculated as described in Example 4.

Pre-caecal protein digestibility was ca. 80% in control (pancreatic sufficient) minipigs on the diet used. In the untreated PEI minipig, protein digestibility was severely reduced compared to these control values, but enzyme supplementation with pancreatin or the microbial protease strongly improved digestibility, which approached control values (see the results in Table 3 below).

Table 3: Influence of enzyme supplementation on apparent protein digestibility:

| | 0 Enzymes | 6000 U/meal |
|---|---|---|
| Control | 81.3 ± 2.6 | - |
| PEI untreated | 30.5 ± 6.8 | - |
| PEI + pancreatin | - | 71.5 ± 4.1 |
| PEI + Protease | - | 65.3 ± 0.7 |
| Values are mean ± SD | | |

## Claims

1. An acid stable protease of at least 90% identity to SEQ ID NO: 1, for use as a medicament for treatment of pancreatic exocrine insufficiency, wherein acid stable means that the protease activity of the pure protease enzyme, in a dilution corresponding to $A_{280}$ = 1.0, and following incubation for 2 hours at 37°C in the following buffer: 100mM succinic acid, 100mM HEPES, 100mM CHES, 100mM CABS, 1mM $CaCl_2$, 150mM KCl, 0.01% Triton® X-100, pH 3.5; is at least 80% of the reference activity.

2. The protease of claim 1, in combination with a lipase or an amylase, for use as a medicament.

3. The protease of claim 1, in combination with a lipase and an amylase, for use as a medicament.

4. Use of a protease as defined in claim 1 for the manufacture of a medicament for the treatment of pancreatic exocrine insufficiency.

5. The use of claim 4, further comprising the use of a lipase or an amylase.

6. The use of claim 4, further comprising the use of a lipase and an amylase.

7. A pharmaceutical composition comprising a protease as defined in claim 1, preferably in the form of a solid concentrate, together with at least one pharmaceutically acceptable auxiliary material, for the treatment of pancreatic exocrine insufficiency.

8. The composition of claim 7, further comprising a lipase or an amylase, preferably in the form of a solid concentrate.

9. The composition of claim 7, further comprising a lipase and an amylase, preferably in the form of solid concentrates.

10. The protease of claim 1 for use in the treatment of pancreatic exocrine insufficiency.

11. The protease of claim 10, further comprising the use of a lipase or an amylase.

12. The protease of claim 10, further comprising the use of a lipase and an amylase.


**Patentansprüche**

1. Säurestabile Protease von mindestens 90 % Identität zu SEQ ID NO: 1 zur Verwendung als ein Medikament zur Behandlung von exokriner Pankreasinsuffizienz, wobei säurestabil bedeutet, dass die Proteaseaktivität des reinen Proteaseenzyms, in einer Verdünnung, entsprechend zu $A_{280}$ = 1,0, und anschließend Inkubation für 2 Stunden bei 37 °C in dem folgenden Puffer: 100 mM Bernsteinsäure, 100 mM HEPES, 100 mM CHES, 100 mM CABS, 1 mM $CaCl_2$, 150 mM KCl, 0,01 % Triton® X-100, pH 3,5; mindestens 80 % von der Referenzaktivität beträgt.

2. Protease nach Anspruch 1, in Kombination mit einer Lipase oder Amylase zur Verwendung als ein Medikament.

3. Protease nach Anspruch 1, in Kombination mit einer Lipase und einer Amylase, zur Verwendung als ein Medikament.

4. Verwendung einer wie in Anspruch 1 definierten Protease für die Herstellung eines Medikaments für die Behandlung von exokriner Pankreasinsuffizienz.

5. Verwendung nach Anspruch 4, des Weiteren umfassend die Verwendung einer Lipase oder einer Amylase.

6. Die Verwendung nach Anspruch 4, des Weiteren umfassend die Verwendung einer Lipase und einer Amylase.

7. Pharmazeutische Zusammensetzung, umfassend eine wie in Anspruch 1 definierte Protease, vorzugsweise in der Form eines Feststoffkonzentrats, zusammen mit mindestens einem pharmazeutisch verträglichen Hilfsmaterial, für die Behandlung von exokriner Pankreasinsuffizienz.

8. Zusammensetzung nach Anspruch 7, des Weiteren umfassend eine Lipase oder eine Amylase, vorzugsweise in der Form eines Feststoffkonzentrats.

9. Zusammensetzung nach Anspruch 7, des Weiteren umfassend eine Lipase und eine Amylase, vorzugsweise in der Form von Feststoffkonzentraten.

**10.** Protease nach Anspruch 1 zur Verwendung in der Behandlung von exokriner Pankreasinsuffizienz.

**11.** Protease nach Anspruch 10, des Weiteren umfassend die Verwendung einer Lipase oder einer Amylase.

**12.** Protease nach Anspruch 10, des Weiteren umfassend die Verwendung einer Lipase und einer Amylase.

**Revendications**

**1.** Protéase stable dans des acides d'au moins 90% d'identité avec SEQ ID NO: 1, pour une utilisation comme médicament pour le traitement de l'insuffisance pancréatique exocrine, dans laquelle « stable dans des acides » signifie que l'activité protéase de l'enzyme protéase pure, à une dilution correspondant à $A_{280} = 1,0$, et suivant une incubation de 2 heures à 37°C dans le tampon suivant : 100mM d'acide succinique, 100mM d'HEPES, 100mM de CHES, 100mM de CABS, 1mM de CaC12, 150mM de KCl, 0,01% de Triton® X-100, pH 3.5 ; est d'au moins 80% de l'activité de référence.

**2.** Protéase de la revendication 1, en combinaison avec une lipase ou une amylase, pour une utilisation comme médicament.

**3.** Protéase de la revendication 1, en combinaison avec une lipase et une amylase pour une utilisation comme médicament.

**4.** Utilisation d'une protéase telle que définie dans la revendication 1 pour la fabrication d'un médicament pour le traitement de l'insuffisance pancréatique exocrine.

**5.** Utilisation de la revendication 4, comprenant en outre l'utilisation d'une lipase ou d'une amylase.

**6.** Utilisation de la revendication 4, comprenant en outre l'utilisation d'une lipase et d'une amylase.

**7.** Composition pharmaceutique comprenant une protéase telle que définie dans la revendication 1, de préférence sous forme d'un concentré solide, avec au moins un matériau auxiliaire pharmaceutiquement acceptable, pour le traitement d'une insuffisance pancréatique exocrine.

**8.** Composition de la revendication 7, comprenant en outre une lipase ou une amylase, de préférence sous forme d'un concentré solide.

**9.** Composition de la revendication 7, comprenant en outre une lipase et une amylase, de préférence sous forme de concentrés solides.

**10.** Protéase de la revendication 1 utile pour le traitement d'une insuffisance pancréatique exocrine

**11.** Protéase de la revendication 10, comprenant en outre l'utilisation d'une lipase ou d'une amylase.

**12.** Protéase de la revendication 10, comprenant en outre d'utilisation d'une lipase et d'une amylase.

# EP 1 755 656 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5614189 A
- EP 600868 A
- WO 0054799 A
- WO 02060474 A
- WO 8803947 A
- WO 0158276 A
- WO 2004111220 A
- WO 2004111221 A
- WO 2004111222 A
- WO 2004111223 A
- WO 2005035747 A
- WO 2004111219 A
- EP 305216 A
- WO 9205249 A
- WO 9219726 A
- WO 9425577 A
- WO 9522615 A
- WO 9704079 A
- WO 9707202 A
- WO 9942566 A
- WO 0032758 A
- WO 0060063 A
- WO 0183770 A
- WO 02055679 A
- WO 02066622 A
- EP 785994 A
- EP 869167 A
- EP 652945 A
- WO 8901969 A
- WO 0134784 A
- WO 9919467 A
- US 4933279 A
- EP 722490 A
- EP 904360 A
- WO 9617929 A
- WO 9830682 A
- WO 9835026 A
- WO 9900489 A
- WO 0026354 A
- WO 9616177 A
- WO 0026230 A
- WO 0183559 A
- EP 561907 A
- WO 0022103 A
- EP 691982 A
- EP 0600868 A

### Non-patent literature cited in the description

- *Eur. J. Biochem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- Handbook of Proteolytic Enzymes. Academic Press, 1998
- *Biochem.J.,* 1993, vol. 290, 205-218
- **Rawlings, N.D. ; O'Brien, E. A. ; Barrett, A.J.** MEROPS: the protease database. *Nucleic Acids Res.,* 2002, vol. 30, 343-346
- **Uppenberg et al.** *Structure,* 1994, vol. 2, 293
- **W. R. Pearson ; D. J. Lipman.** Improved Tools for Biological Sequence Analysis. *PNAS,* 1988, vol. 85, 2444-2448
- **W. R. Pearson.** Rapid and Sensitive Sequence Comparison with FASTP and FASTA. *Methods in Enzymology,* 1990, vol. 183, 63-98
- **T. F. Smith ; M. S. Waterman.** Smith-Waterman algorithm. *J. Mol. Biol.,* 1981, vol. 147, 195-197
- **Kato et al.** *J. Biol. Chem.,* 1991, vol. 266, 3361
- *JOP. J Pancreas,* 2002, vol. 3 (5), 116-125
- **S.C.Gill ; P.H. von Hippel.** *Analytical Biochemistry,* 1989, vol. 182, 319-326
- A.O.A.C. (1984) Official Methods of Analysis. 1984
- **Savoie,L. ; Gauthier,S.F.** Dialysis Cell For The In-vitro Measurement Of Protein Digestibility. *J. Food Sci.,* 1986, vol. 51, 494-498
- **Sabinszky,L. ; Van,D.M.J.M. ; Boer,H. ; Den,H.L.A.** An In-vitro Method for Prediction of The Digestible Crude Protein Content in Pig Feeds. *J. Sci. Food Agr.,* 1990, vol. 50, 173-178
- **Boisen,S. ; Eggum,B.O.** Critical Evaluation of In-vitro Methods for Estimating Digestibility in Simple-Stomach Animals. *Nutrition Research Reviews,* 1991, vol. 4, 141-162
- Pharmaceutical Enzymes. 1978
- **Deemester et al.** Pharmaceutical Enzymes. Marcel Dekker, 1997, 343-385

- **Tabeling et al.** Studies on nutrient digestibilities (pre-caecal and total) in pancreatic duct-ligated pigs and the effects of enzyme substitution. *J. Anim. Physiol. A. Anim. Nutr.,* 1999, vol. 82, 251-263

- Growth and digestion in pancreatic duct ligated pigs, Effect of enzyme supplementation. **Gregory et al.** Biology of the Pancreas in Growing Animals. Elsevier Science BV, 1999, 381-393
- Animal Nutrition. Longman Scientific and Technical, 1988